# EUROPÄISCHE PATENTANMELDUNG

(11) **EP 0 901 985 A2**
(43) Veröffentlichungstag der Anmeldung: **17.03.1999**
(21) Anmeldenummer: 98116070.8
(22) Anmeldetag: 26.08.1998
(51) Int. Cl.: C01B 23/00, B01D 53/00

(54) **Abtrennung von Xenon aus Gasgemischen**

(30) Priorität: 13.09.1997 DE 19740280
(71) Anmelder: MESSER GRIESHEIM GMBH, 60547 Frankfurt (DE)
(72) Erfinder: Hamm, Reiner, Dr., 47506 Neukirchen-Vluyn (DE)

(57) **Zusammenfassung**

Das Verfahren zur Abtrennung von Xenon aus einem Xenon-haltigen Narkosegas oder ausgeatmetem Xenon-haltigen Gasgemisch basiert auf einer barotropen Trennung. Das Narkosegas oder das Gasgemisch wird mit einer Flüssigkeit (z. B. Wasser) vermischt und verdichtet, so daß sich Xenon in einer unteren Phase abscheidet.

## Beschreibung

Die Erfindung betrifft ein Verfahren zur Abtrennung von Xenon aus einem Gasgemisch.

Die narkotischen Wirkungen von Xenon sind seit den 40er Jahren bekannt. Eine Mischung von etwa 80% Xenon und 20% Sauerstoff wird als nahezu ideales Narkosegas betrachtet, das gegenüber den heute überwiegend verwendeten Narkosegasen auf der Basis von Lachgas zahlreiche Vorteile hat. Wegen der hohen Kosten von Xenon werden Xenon-Narkosen im klinischen Bereich praktisch nicht angewandt. Um diese Kosten zu verringern, wurden daher z.B. in DE 44 11 533 C1 Narkosegeräte mit einer Rückgewinnungsanlage für Xenon vorgeschlagen. Bei der in DE 44 11 533 C1 vorgeschlagenen Rückgewinnungsanlage wird das ausgeatmete Atemgas nach einer Vorreinigung komprimiert und in einen Druckbehälter eingeleitet, welcher in eine Kühlvorrichtung aufgenommen ist. Über die Kühlvorrichtung wird der Druckbehälter so weit abgekühlt, daß das rückzugewinnende Xenon sich verflüssigt. Die gasförmigen Bestandteile in dem Druckbehälter werden über eine Abströmdrossel abgelassen. Wenn eine genügende Menge an flüssigem Xenon in dem Druckbehälter vorhanden ist, wird dieses in einen weiteren Behälter umgepumpt. Diese Vorrichtung hat den Nachteil eines hohen apparativen Aufwands für das Komprimieren des Gases und das Abkühlen eines ganzen Behälters. Außerdem ist der Transfergrad der Xenonrückgewinnung nicht zufriedenstellend.

DE 41 08 653 A1 beschreibt ein Verfahren zur Trennung verdichteteter Gase verschiedener Molekulargewichte unter Ausnutzung der Umkehr der Massendichte im Vergleich zu einer Flüssigkeit. Es wird sehr allgemein ein Trennprinzip beschrieben, ohne zur Trennung geeignete Gasgemische zu nennen.

Es ist die Aufgabe der Erfindung, ein einfaches und wirtschaftliches Verfahren zum Abtrennen von Xenon aus dem exspiratorischen Atemgas eines Narkosepatienten zu schaffen. Eine weitere Aufgabe der Erfindung besteht darin, einen wirtschaftlichen Recyclingprozeß für ein Narkosegas auf Xenonbasis, zu schaffen.

Diese Aufgaben werden durch ein Verfahren zur Abtrennung von Xenon aus einem Xenon-haltigen Narkosegas oder ausgeatmetem Xenon-haltigen Gasgemisch gelöst, wobei das Narkosegas oder das Gasgemisch mit einer Flüssigkeit vermischt und verdichtet wird, so daß sich Xenon in einer unteren Phase abscheidet.

Als Flüssigkeit eignet sich beispielsweise Wasser, Ammoniak, Pentan oder Heptan.

Das Prinzip und die Durchführung des Verfahrens ist allgemein in DE 41 08 653 A1 beschrieben, worauf hiermit Bezug genommen wird.

Bei dem Verfahren wird das Gasgemisch mit der Flüssigkeit soweit komprimiert, daß das Xenon bereits überbarotrop ist. Die Löslichkeit von Xenon in der Flüssigkeit muß größer sein als die Löslichkeit der anderen Gaskomponenten. Eine Übersättigung der Flüssigkeit mit Xenon wird z. B. durch eine weitere Druckerhöhung erreicht. Bei Entspannung des Druckes auf einen Druck, bei dem das Xenon ein höheres spezifisches Gewicht als die Flüssigkeit aufweist (barotrope Bedingungen), scheidet sich Xenon in einer unteren Phase ab.

Das Verfahren kann auch so ausgeführt werden, daß bei einer Temperatur das Xenon bevorzugt in der Flüssigkeit gelöst wird und durch Temperaturerhöhung eine Übersättigung erzielt wird.

Ist die verwendete Flüssigkeit Wasser, dann kann die Bildung von Xenon-Hydrat ausgenutzt werden. Das Xenon-Hydrat ist spezifisch schwerer und fällt aus. Das Xenon-Hydrat absorbiert auch in der Regel Xenon. Wenn durch Änderung von Temperatur oder Druck das Xenon-Hydrat schmilzt, wird das Xenon freigesetzt. Wenn barotrope Bedingungen noch vorliegen, bildet sich unterhalb der Wasserphase eine Phase mit Xenon.

Das Verfahren eignet sich insbesondere zur Trennung von Gasgemischen, enthaltend Xenon, Stickstoff und Sauerstoff.
Das Expirationsgas eines Narkosepatienten enthält als Hauptkomponenten Xenon, Stickstoff, Sauerstoff und Kohlendioxid. Zur Abtrennung von Kohlendioxid wird das Exspirationsgas durch einen Filter oder Absorber ( z. B. mit Atemkalk als Absorbermasse) geleitet oder einer Gaswäsche (z. B. Lauge) unterzogen. Gegebenenfalls können organische Verunreinigungen im Gasgemisch mittels Aktivkohle entfernt werden.

Das zu trennende, vorzugsweise vorgereinigte Gasgemisch wird in einen Hochdruckbehälter (Autoklav) eingeleitet, der zuvor teilweise mit der Trennflüssigkeit, bevorzugt Wasser und besonders bevorzugt destilliertes Wasser, befüllt wurde. Der Druck wird über den Barotropie-Grenzdruck erhöht. Die Temperatur wird so gewählt, daß Xenon-Hydrat ausfällt, das zu Boden sinkt. Durch Temperatur-Erhöhung und Druckemiedrigung dissoziiert das Xenon-Hydrat. Bleibt man über dem Grenzdruck, so wird durch Barotropie die Wasserschicht angehoben und trennt Xenon (unten) vom Sauerstoff und Stickstoff im Gemisch (oben).

Das Xenon-Hydrat kann durch Temperatur- und/oder Druckänderung in Xenon und Wasserdampf gespalten werden. Beispielsweise kann man durch Ablassen von einem Teil der überstehenden Gasphase (Leichtgas) den Druck etwas absenken; jedoch muß darauf geachtet werden, daß man dabei noch über dem Barotropie-Grenzdruck bleibt.

Das bei der Dissoziation des Xenon-Hydrats entstehende feuchte (überkritische) Xenon kann über eine Trocknerpatrone (z. B. Filterpatrone mit Silicagel) geleitet und dann zur weiteren Verwendung abgeführt werden. Wenn das rückgewonnene Xenon direkt wieder dem Narkosegerät zugeleitet wird, entfällt die Trocknung.

Der Barotropie-Grenzdruck ist jener Druck, bei dem die Dichte des komprimierten Xenons gleich der Dichte der Trennflüssigkeit wird, z. B. gleich der Dichte des Wassers. Der Grenzdruck ist eine Funktion der Temperatur und beträgt bei Verwendung von Wasser z. B. bei 37 °C 79 bar. Am kritischen Punkt des Xenons (16,6 °C / 58,4 bar) sowie im Punkt (50 °C / 100 bar) schichtet sich das Wasser über das Xenon, d. h. man liegt bei diesen Betriebsbedingungen bereits über dem jeweiligen barotropen Punkt.

Zur Verbesserung der Abtrennung von Xenon und zur Gewinnung von Xenon mit erhöhter Reinheit kann das Verfahren in einer Kaskade, d. h. in zwei oder mehreren hintereinandergeschalteten Trennstufen, durchgeführt werden.

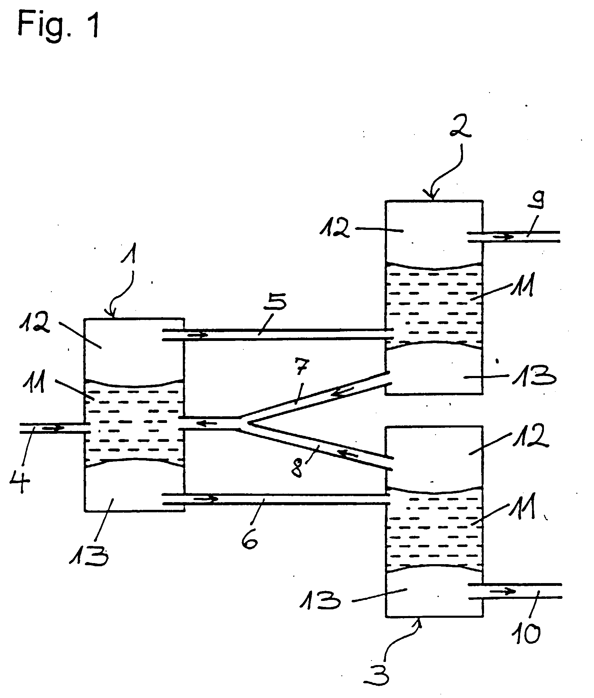

Fig. 1 zeigt schematisch einen kontinuierlichen zweistufigen Prozess mit Rückführung Xenon-haltiger Gasfraktionen, die Xenon nicht als Hauptkomponente enthalten.

Aus dem Behälter (1) wird aus der oberen Gasphase (12) das Gasgemisch (5) entnommen, das aus hauptsächlich Leichtgas (Sauerstoff, Stickstoff) und etwas Xenon besteht, und dem Behälter (2) zugeführt. Aus der unteren Gasphase (13) von Behälter (1) wird das Gasgemisch (6) entnommen, das neben Xenon als Hauptkomponente noch etwas Leichtgas enthält, und dem Behälter (3) zugeführt. In den Behältern (2) und (3) werden die Gase nochmals barotrop aufgetrennt. Das Schwergas (7)(untere Gasphase (13)) aus dem Behälter (2) und das Leichtgas (8)(obere Gasphase (12)) aus dem Behälter (3) werden zurückgeführt in den Behälter (1), wobei die Einspeisung in die Trennflüssigkeit (11) (z. B. Wasser) erfolgt. Das aus dem Behälter (2) entnommene Gas (9) mit Leichtgas wird in der Regel verworfen. Das aus dem Behälter (3) entnommene Gas (10) ist das zurückgewonnene, reine Xenon (Schwergas). Das zurückgewonnene Xenon kann entweder in einen Druckgasbehälter verdichtet werden oder mit geeignetem Druck wieder dem Narkosegerät zugeführt werden. Sollte das gewonnene Xenon nach zwei Trennstufen immer noch nicht genügend rein sein, so kann der Prozess auf drei oder mehrere Trennstufen erweitert werden.

## Patentansprüche

1. Verfahren zur Abtrennung von Xenon aus einem Xenon-haltigen Narkosegas oder ausgeatmetem Xenon-haltigen Gasgemisch, dadurch gekennzeichnet, daß das Narkosegas oder das Gasgemisch mit einer Flüssigkeit vermischt und verdichtet wird, so daß sich Xenon in einer unteren Phase abscheidet.

2. Verfahren gemäß Anspruch 1, dadurch gekennzeichnet, daß als Flüssigkeit Wasser verwendet wird und Xenon-Hydrat ausfällt.

3. Verfahren gemäß Anspruch 1 oder 2, dadurch gekennzeichnet, daß zur Abscheidung der unteren Phase mit Xenon eine Temperatur im Bereich von 273 K bis 500 K und ein Druck im Bereich von 40 bar bis 300 bar eingesetzt wird.

4. Verfahren gemäß einem der Ansprüche 1 bis 3, dadurch gekennzeichnet, daß zur Abscheidung der unteren Phase mit Xenon eine Temperatur im Bereich von 290 bis 500 K und ein Druck im Bereich von 58 bis 300 bar eingesetzt wird.

5. Verfahren gemäß einem der Ansprüche 1 bis 4, dadurch gekennzeichnet, daß die untere Phase mit Xenon von den übrigen Phasen abgetrennt wird.

6. Verfahren gemäß einem der Ansprüche 1 bis 5, dadurch gekennzeichnet, daß die untere Phase mit Xenon von den übrigen Phasen abgetrennt wird und erneut mit einer Flüssigkeit vermischt und verdichtet wird, so daß sich Xenon in einer unteren Phase abscheidet.

7. Narkosesystem mit einer Vorrichtung zur Abtrennung von Xenon aus einem Xenon-haltigen Narkosegas oder ausgeatmetem Xenon-haltigen Gasgemisch, in der das Narkosegas oder das Gasgemisch mit einer Flüssigkeit vermischt und verdichtet wird, so daß sich Xenon in einer unteren Phase abscheidet.
